# DEMANDE DE BREVET EUROPEEN

(11) **EP 2 392 294 A1**
(43) Date de publication de la demande: **07.12.2011**
(21) Numéro de dépôt: 11168785.1
(22) Date de dépôt: 06.06.2011
(51) Int. Cl.: A61F 2/28, A61F 2/36, A61F 2/40, A61B 17/72

(54) **Prothèse modulaire, et kit chirurgical comprenant au moins une telle prothèse modulaire**

(30) Priorité: 07.06.2010 FR 1054448; 17.12.2010 FR 1004933
(71) Demandeur: Tornier, 38330 Saint-Ismier (FR)
(72) Inventeur: Ratron, Yves-Alain, 38000 GRENOBLE (FR); Ferrari, Irène, 38660 LE TOUVET (FR)
(74) Mandataire: Grand, Guillaume

(57) **Abrégé**

La présente invention concerne une prothèse modulaire (300), comprenant au moins un premier élément (310) et un deuxième élément (320), caractérisée en ce qu'au moins une chambre de réception (327) d'un fluide est délimitée à l'interface entre le premier élément (310) et le deuxième élément (320). L'invention concerne aussi un kit chirurgical, caractérisé en ce qu'il comprend au moins une prothèse modulaire (300) selon l'une des revendications précédentes, avec au moins un premier élément (310) et au moins deux deuxièmes éléments (320), chaque deuxième élément présentant des dimensions différentes du ou des autres deuxièmes éléments et étant configuré pour former au moins une chambre (327) de dimensions différentes à l'interface avec le ou l'un des premiers éléments.

## Description

La présente invention concerne une prothèse modulaire. L'invention concerne également un kit chirurgical, comprenant au moins une telle prothèse modulaire. Le domaine de l'invention est celui des implants modulaires, notamment pour la réduction des fractures d'épaules.

Il est connu des prothèses monoblocs, qui peuvent être implantées de manière rigide, notamment grâce à l'utilisation de ciment osseux, mais nécessitent l'utilisation d'implants d'essai et présentent des difficultés de réglage. Il est également connu des prothèses modulaires, qui offrent une grande souplesse de réglage mais présentent plusieurs inconvénients, comme la fragilité, le risque de casse, et le risque de déplacements relatifs entre éléments modulaires.

WO-A-03/034954 et US-A-5 340 362 décrivent chacun une prothèse osseuse intra-médullaire rigidement fixée en position dans la cavité osseuse par injection de ciment.

US-A-2003/0074078 décrit une prothèse modulaire ayant un élément de tige intra-médullaire qui est inséré dans l'os. Un trochanter artificiel est disposé sur la tige, et un col est disposé sur le trochanter. Le positionnement relatif des différents éléments modulaires, en particulier le réglage en hauteur de la prothèse, est effectué par la mise en oeuvre d'une pièce distale filetée qui est rapportée sur la tige, avec un manchon intermédiaire disposé entre la pièce distale et la tige. Une vis de calage et un boulon permettent d'ajuster le positionnement relatif des différents éléments et de verrouiller la prothèse avec la hauteur désirée. Toutefois, cette prothèse présente un grand nombre d'éléments modulaires, ce qui, d'une part, la rend complexe à mettre en oeuvre par le chirurgien et, d'autre part, augmente le risque de casse et de déplacement relatif entre éléments. Ainsi, cette prothèse ne présente pas un fonctionnement satisfaisant.

US-A-5 466 261 décrit une prothèse modulaire destinée aux patients juvéniles, comme substitut pour les os ayant été endommagés par la maladie ou un traumatisme. La prothèse est ajustable alors que le patient grandit. Dans ces conditions, la prothèse n'est pas adaptée pour recevoir tout type de fluide ou ciment et ne présente pas une rigidité de fixation satisfaisante.

Le but de la présente invention est de proposer une prothèse présentant conjointement une souplesse de réglage et une rigidité de fixation.

A cet effet, l'invention concerne une prothèse modulaire, comprenant au moins un premier élément et un deuxième élément, caractérisée en ce qu'au moins une chambre de réception d'un fluide est délimitée à l'interface entre le premier élément et le deuxième élément.

Ainsi, le chirurgien peut mettre en place le premier élément, qui est notamment un élément diaphysaire, puis régler de manière simple et précise la position relative du deuxième élément, qui est alors un élément métaphysaire, par rapport au premier élément. Chacune des chambres délimitées à l'interface des éléments est adaptée pour recevoir un fluide, qui peut être un fluide agglutinant destiné à leur verrouillage en position. En étant délimitée directement à l'interface entre le premier élément et le deuxième élément, la chambre de réception recevant du fluide agglutinant permet un verrouillage en position rigide et sécurisé de la prothèse. En alternative, un fluide peut être introduit dans la chambre pour écarter le deuxième élément par rapport au premier élément, par exemple dans le cadre d'une reprise chirurgicale.

La prothèse selon l'invention comporte un nombre réduit d'éléments modulaires, est relativement simple à mettre en oeuvre et offre une grande latitude pour le réglage. Cette prothèse supprime les inconvénients inhérents, d'une part, à la mise en place d'une prothèse monobloc, tels que les difficultés de réglage et, d'autre part, à l'usage d'une prothèse modulaire, tels que la fragilité, le risque de casse, et le risque de déplacement relatif entre les éléments modulaires.

Selon d'autres caractéristiques avantageuses de l'invention, prises isolément ou en combinaison :
- Chaque chambre de réception présente un volume variable, notamment en fonction de la quantité de fluide admise dans la chambre.
- Plusieurs chambres de réception isolées sont délimitées à l'intérieur de la prothèse, ces chambres de réception étant aptes à être remplies indépendamment les unes des autres.
- Le positionnement relatif entre le premier élément et le deuxième élément est variable axialement suivant un axe longitudinal de la prothèse et/ou transversalement à cet axe longitudinal et/ou angulairement autour de cet axe longitudinal.
- L'un des éléments parmi le premier élément et le deuxième élément inclut au moins un canal d'injection pour l'injection du fluide dans la ou les chambres de réception.
- Le deuxième élément inclut au moins une cavité de couplage apte à recevoir au moins partiellement le premier élément, et en ce que la ou les chambres de réception sont délimitées dans la cavité de couplage.
- Une partie proximale du premier élément et la cavité de couplage du deuxième élément comprennent des moyens de blocage complémentaires, qui sont aptes à coopérer et bloquer le déplacement relatif en rotation entre le premier élément et le deuxième élément, notamment des rainures, cannelures, dentures et/ou aspérités de surface.
- L'un des éléments parmi le premier élément et le deuxième élément inclut au moins un canal d'injection pour l'injection du fluide dans la ou les chambres de réception, et le canal d'injection, la cavité de couplage et le premier élément sont coaxiaux.
- Un épaulement est agencé entre une partie distale et une partie proximale du premier élément.
- L'un des éléments parmi le premier élément et le deuxième élément inclut une gorge de réception annulaire, du côté de la ou les chambres de réception qui est orienté vers l'autre élément, et en ce qu'un joint d'étanchéité annulaire est disposé dans la gorge de réception de manière à réaliser l'étanchéité de la ou des chambres de réception de fluide du côté tourné vers l'autre élément.
- La ou au moins l'une des chambres de réception est configurée pour recevoir un fluide agglutinant à l'interface entre le premier élément et le deuxième élément, notamment au contact direct du premier élément et du deuxième élément.
- La prothèse comprend un fluide agglutinant remplissant la ou l'une des chambres de réception.
- Le fluide est un fluide agglutinant choisi parmi les matériaux suivants : polyméthacrylate de méthyle, résine bio-compatible, matériau adhésif, matériau polymère, ciment chirurgical, substitut osseux injectable, et de préférence le temps de séchage du fluide agglutinant est compris entre 5 et 30 minutes.
- Le fluide est non-agglutinant et choisi parmi de l'air, de l'eau, une solution liquide ou un gel aqueux.

L'invention a également pour objet un kit chirurgical comprenant au moins une prothèse modulaire telle que mentionnée ci-dessus, avec au moins un premier élément et au moins deux deuxièmes éléments, chaque deuxième élément présentant des dimensions différentes du ou des autres deuxièmes éléments et étant configuré pour former au moins une chambre de dimensions différentes à l'interface avec le ou l'un des premiers éléments.

Par ailleurs, une méthode chirurgicale d'implantation d'une prothèse modulaire telle que mentionnée ci-dessus est décrite ci-après. La méthode comprend au moins les étapes suivantes :
a) le chirurgien prépare une cavité osseuse dans un os nécessitant une réparation ;
b) le chirurgien dispose dans l'os le premier élément, qui est un élément diaphysaire allongé comprenant une partie distale qui est fixée dans la cavité osseuse à l'aide de ciment chirurgical et une partie proximale qui reste en saillie à l'extérieur de l'os;
c) le chirurgien dispose le deuxième élément, qui est un élément métaphysaire, sur la partie proximale libre de l'élément diaphysaire, ces éléments étant mobiles relativement l'un à l'autre, et
d) le chirurgien règle la position de la prothèse en injectant progressivement un fluide à l'interface de l'élément métaphysaire et de l'élément diaphysaire.

Selon d'autres caractéristiques de cette méthode chirurgicale, prises isolément ou en combinaison :
- Dans l'étape d), le fluide est un fluide agglutinant, et la méthode comprend également une étape e) dans laquelle l'élément métaphysaire et l'élément diaphysaire sont immobilisés rigidement l'un par rapport à l'autre lors du séchage du fluide agglutinant.
- Dans l'étape c), la partie proximale de l'élément diaphysaire pénètre dans une cavité de couplage qui est incluse dans l'élément métaphysaire, en délimitant au moins une chambre de réception de fluide dans la cavité de couplage, à l'interface entre l'élément diaphysaire et l'élément métaphysaire.
- Chaque chambre de réception présente un volume variable, notamment en fonction de la quantité de fluide admise dans la chambre.
- Dans l'étape d), le fluide est injecté à l'interface de l'élément métaphysaire et de l'élément diaphysaire, le cas échéant dans l'une des chambres de réception, par un canal d'injection qui est inclus dans l'élément métaphysaire.

En outre, est décrite une méthode chirurgicale d'utilisation d'unela prothèse modulaire telle que mentionnée ci-dessus, en particulier au cours d'une reprise de prothèse, consistant à désolidariser le premier élément et le deuxième élément en introduisant du fluide sous pression dans l'une des chambres de réception.

L'invention sera mieux comprise à la lecture de la description qui va suivre, donnée uniquement à titre d'exemple non limitatif et faite en se référant aux dessins sur lesquels :
- la figure 1 est une coupe d'une prothèse modulaire conforme à l'invention implantée dans un os fracturé ;
- la figure 2 est une coupe analogue à la figure 1 d'une prothèse modulaire conforme à un deuxième mode de réalisation de l'invention ;
- la figure 3 est une vue en élévation d'une prothèse modulaire conforme à un troisième mode de réalisation de l'invention, montrant une variante d'élément diaphysaire, l'élément diaphysaire n'étant pas représenté ;
- la figure 4 est une coupe analogue à la figure 1 d'une prothèse modulaire conforme à un quatrième mode de réalisation de l'invention ; et
- la figure 5 est une vue à plus grand échelle du détail V à la figure 4 ; et
- la figure 6 est une vue analogue à la figure 5, montrant la prothèse modulaire dans une autre configuration.

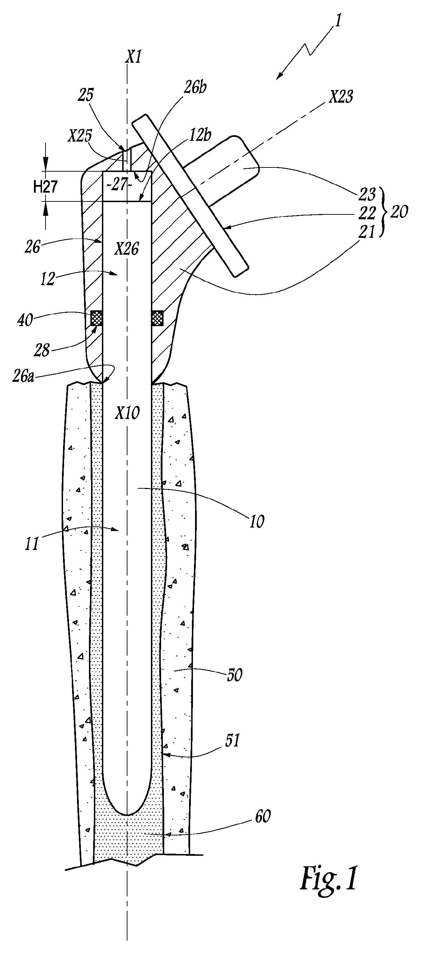

Sur la figure 1 est représentée une prothèse modulaire 1 qui s'étend sensiblement selon un axe X1, comprenant un élément diaphysaire 10 et un élément métaphysaire 20.

La prothèse 1 est destinée à être implantée dans une cavité intramédullaire 51, qui est préparée par le chirurgien dans un os 50. En particulier, l'os est une diaphyse humérale 50, et le chirurgien met en oeuvre la prothèse 1 dans le but de réparer une fracture d'épaule.

L'élément diaphysaire est une tige cylindrique 10 comprenant une partie distale 11 et une partie proximale 12, qui s'étend selon un axe longitudinal X10. Lors de l'implantation de la prothèse 1 dans la diaphyse 50, la tige 10 est tout d'abord insérée dans la cavité diaphysaire 51. Afin de bloquer la tige 10 dans sa configuration finale, on injecte du ciment ou de la résine 60 à l'état liquide dans la cavité 51. Ainsi, après durcissement dudit ciment ou de la dite résine, la partie distale 11 de la tige 10 est fixée rigidement dans la cavité 51 de la diaphyse 50, tandis que la partie proximale 12 est en saillie hors de la cavité 51. Du côté opposé à la cavité 51, la partie proximale 12 présente une extrémité proximale 12b en forme de disque.

En variante non représentée, l'élément diaphysaire 10 peut être un clou, par exemple lors de la révision d'un clou intramédullaire.

L'élément métaphysaire 20 comprend une tête 21, une surface d'appui 22, et un plot 23. La surface d'appui 22 et le plot 23 font office de support pour une articulation sphérique ou une rotule, non représentée. Le plot 23 est de forme cylindrique, et s'étend depuis la surface d'appui 22, perpendiculairement à celle-ci, selon un axe X23.

L'élément métaphysaire 20 est fabriqué par des techniques connues de moulage, usinage et/ou perçage. L'élément métaphysaire 20 représenté sur la figure 1 est monobloc.

En variante non représentée, la surface d'appui 22 et le plot 23 peuvent être rapportés sur la tête 21.

La tête 21 est partiellement creuse et inclut : un canal d'injection 25 présentant un axe longitudinal X25, une cavité de couplage 26 présentant un axe longitudinal X26 et dans laquelle peut être délimitée une chambre de réception 27 de fluide, et une gorge de réception 28 d'un joint annulaire 40. Le canal d'injection 25 et la cavité 26 sont cylindriques. Pour certaines applications, comme visible sur la figure 1, le canal d'injection 25 et la cavité 26 sont coaxiaux. Cela permet par exemple de faciliter leur réalisation dans la tête 21, notamment par perçage. La cavité 26 présente une embouchure ouverte 26a du côté de la cavité diaphysaire 51, ainsi qu'une paroi interne 26b qui s'étend perpendiculairement et coaxialement à l'axe X26 et au niveau de laquelle débouche le canal 25.

En variante non représentée, le canal d'injection 25 et la cavité 26 ne sont pas coaxiaux.

En pratique, l'élément métaphysaire 20 est positionné par le chirurgien sur la tige 10, qui fait office de support intermédiaire entre l'élément métaphysaire 20 et l'os 50. Plus précisément, la partie proximale 12 de la tige 10 pénètre dans la cavité de couplage 26, avec coulissement des surfaces cylindriques complémentaires. Ainsi, la chambre 27, qui est délimitée par les parois cylindriques de la cavité 26, la surface 26b et l'extrémité 12b de la partie proximale 12, présente un volume variable. En particulier, la chambre de réception 27 présente une dimension H27 variable suivant la direction longitudinale de la prothèse 1, définie par son axe X1, lequel est confondu avec l'axe X10 de la tige 10. Cette dimension H27 peut être mesurée suivant l'axe X1 entre les surfaces 12b et 26b.

A ce stade, la hauteur de la prothèse 1 peut être réglée en injectant progressivement un fluide, non représenté, par le canal d'injection 25, jusque dans la chambre de réception 27. Le fluide est injecté à l'interface entre la tige 10 et l'élément 20, à leur contact direct, à l'exclusion de tout autre élément présent dans la chambre 27. La tige 10 est rigidement fixée dans la cavité diaphysaire 51, tandis que l'élément métaphysaire 20 peut se déplacer relativement à la partie proximale 12. Sous l'effet de la pression interne à la chambre 27, exercée par le fluide entre la cavité 26 et la partie proximale 12, le volume de la chambre 27 augmente. Plus précisément, la dimension H27 de la chambre de réception 27 de fluide est variable, en fonction de la quantité de fluide admise dans la chambre 27, suivant l'axe X1. De ce fait, lorsque le fluide est injecté, la hauteur de la prothèse 1 augmente progressivement jusqu'à atteindre la position désirée.

En variante non représentée, l'élément métaphysaire 20 est pourvu de moyens de visualisation de la hauteur de la prothèse, par exemple des graduations.

Selon une variante préférée, le fluide peut être un fluide agglutinant. Ainsi, lors de la prise du fluide agglutinant, en particulier son séchage, les éléments modulaires 10 et 20 sont bloqués entre eux dans la position choisie, en translation et en rotation, avec une hauteur bien maîtrisée. En d'autres termes, la conception particulière de la prothèse modulaire 1 et l'injection de fluide agglutinant permettent le réglage télescopique de la hauteur. De plus, le positionnement angulaire relatif entre les éléments 10 et 20, autour de l'axe longitudinal X1, est variable et peut être réglé avec précision, avant, pendant ou après l'injection du fluide agglutinant.

Par fluide agglutinant, on entend une matière susceptible d'agglutiner entre eux les éléments avec lesquels il se trouve en contact, c'est-à-dire les réunir en un ensemble monobloc, les solidariser pour empêcher tout mouvement relatif. Cette matière est fluide lors de son introduction dans la chambre de réception, puis sèche progressivement jusqu'à se rigidifier, afin de présenter un volume solide durci une fois la prothèse implantée.

De préférence, le fluide agglutinant est du polyméthacrylate de méthyle (PMMA), une résine biocompatible, un matériau adhésif, un matériau polymère, du ciment chirurgical, ou un substitut osseux injectable. Le fluide agglutinant peut par exemple être initialement contenu dans un récipient déformable comprenant un conduit d'injection, ou être injecté avec un pistolet manuel. Des injections successives peuvent être réalisées jusqu'à ce que la hauteur souhaitée soit atteinte, puis le séchage du fluide agglutinant permet de verrouiller les éléments modulaires entre eux, au niveau de la cavité de couplage 26 et de la chambre de réception 27. Avantageusement, le temps de séchage du fluide agglutinant est compris entre 5 et 30 minutes.

Par ailleurs, un fluide « désagglutinant » ou « fluidifiant » peut être ultérieurement injecté dans la chambre de réception 27. Ce fluide permet de dissoudre ou de refluidifier le fluide agglutinant, afin de modifier le positionnement relatif des éléments de la prothèse, par exemple lors d'une intervention post-opératoire.

La partie proximale 12 de la tige 10 est ajustée dans la cavité 26, toutefois la présence du joint annulaire 40 permet éventuellement de compléter l'étanchéité de la chambre 27 et de la cavité 26 du côté orienté vers la diaphyse 50.

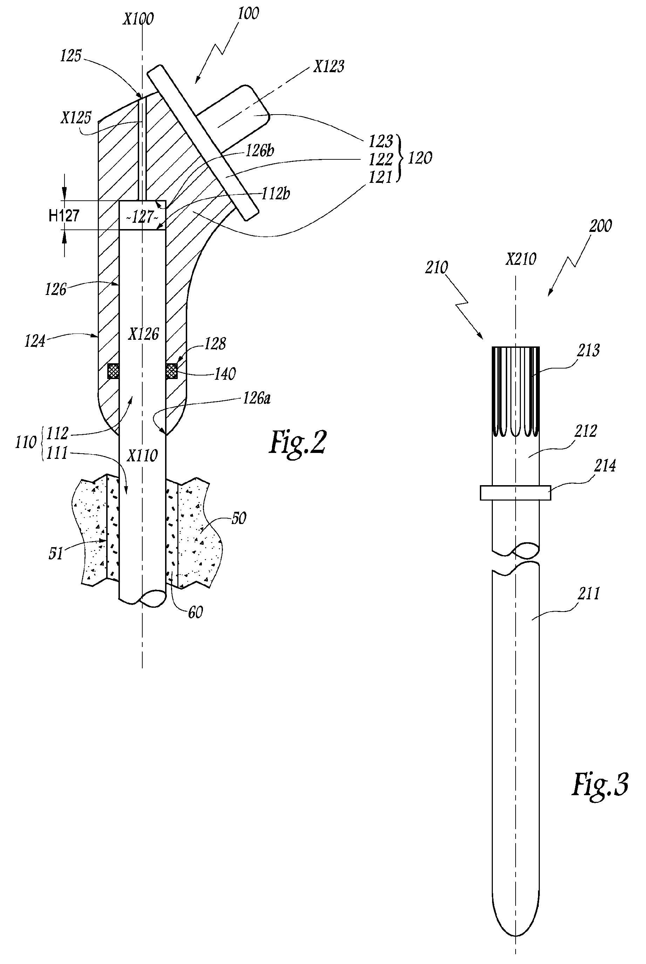

Sur la figure 2 est représenté un deuxième mode de réalisation d'une prothèse modulaire 100 conforme à l'invention. La prothèse 100 est destinée à être disposée dans le même os 50 que dans le premier mode de réalisation.

Certains éléments constitutifs de la prothèse 100 sont similaires aux éléments constitutifs de la prothèse 1 du premier mode de réalisation, décrit plus haut, et portent les mêmes références augmentées de 100. Il s'agit de la tige 110 d'axe X110 comprenant une partie distale 111 et une partie proximale 112 avec une extrémité 112b, de la surface d'appui 122, du plot 123 d'axe X123, du canal d'injection 125 d'axe X125, de la cavité de couplage 126 avec un axe X126, une embouchure 126a et une surface intérieure 126b, de la gorge de réception 128, du joint annulaire 140.

En revanche, les dimensions de l'élément métaphysaire 120 sont différentes des dimensions de l'élément métaphysaire 20 du premier mode de réalisation. Plus précisément, la tête 121 de l'élément métaphysaire 120 présente des dimensions et un agencement interne particuliers, notamment une partie distale 124 allongée dans laquelle s'étendent la cavité de couplage 126 et le canal d'injection 125 allongé. Cette configuration permet, pour une plage de réglage en hauteur identique à la plage de réglage de la première configuration, d'accéder à des dimensions de prothèses différentes de celles de la première configuration. En effet, si la plage de réglage en hauteur de la première configuration se trouve insuffisante pour obtenir le réglage optimal, la seconde configuration sera employée pour obtenir le réglage optimal. Comme dans le premier mode de réalisation, la dimension H127 de la chambre de réception 127 de fluide agglutinant est variable en fonction de la quantité de fluide admise dans la chambre 127.

Ainsi, grâce à la configuration de la chambre 127 et de la partie allongée 124, la hauteur à laquelle la prothèse 100 peut être réglée est supérieure à celle du premier mode de réalisation.

En pratique, une gamme d'éléments métaphysaires 20, 120, et autres, peut couvrir différentes plages de hauteur, de manière à répondre au mieux aux besoins du chirurgien. De préférence, chaque élément métaphysaire couvre approximativement une plage de 15 mm en hauteur.

Sur la figure 3 est représenté un troisième mode de réalisation d'une prothèse modulaire 200 conforme à l'invention, comprenant une variante de tige diaphysaire 210. La tige cylindrique 210 comprend une partie distale 211 et une partie proximale 212, séparées par un épaulement 214. L'élément métaphysaire, la diaphyse et le ciment ne sont pas représentés sur la figure 3.

L'épaulement 214 est destiné à venir en appui contre la surface extérieure de la diaphyse. Ainsi, l'épaulement 214 forme un repère bloquant lors de l'introduction de la tige 210 dans la cavité diaphysaire, assurant une bonne longueur d'enfoncement de la tige 210 dans la cavité diaphysaire.

La partie proximale 212 comprend des rainures longitudinales 213 destinées à venir coopérer avec des rainures correspondantes, non représentées, de la cavité de couplage 26 ou 126 de l'élément métaphysaire 20 ou 120. Ainsi, le réglage du positionnement angulaire et le blocage relatif en rotation sont facilités.

En variante non représentée, la partie proximale 212 peut comprendre tout type de moyens 213 de blocage en rotation, participant au réglage angulaire, par exemple des cannelures, dentures et/ou aspérités de surface. Dans ce cas, la cavité de l'élément métaphysaire comprend des moyens de blocage correspondants.

Selon une autre variante représentée, l'élément diaphysaire 210 peut comprendre une combinaison différente de moyens de blocage en rotation et en translation.

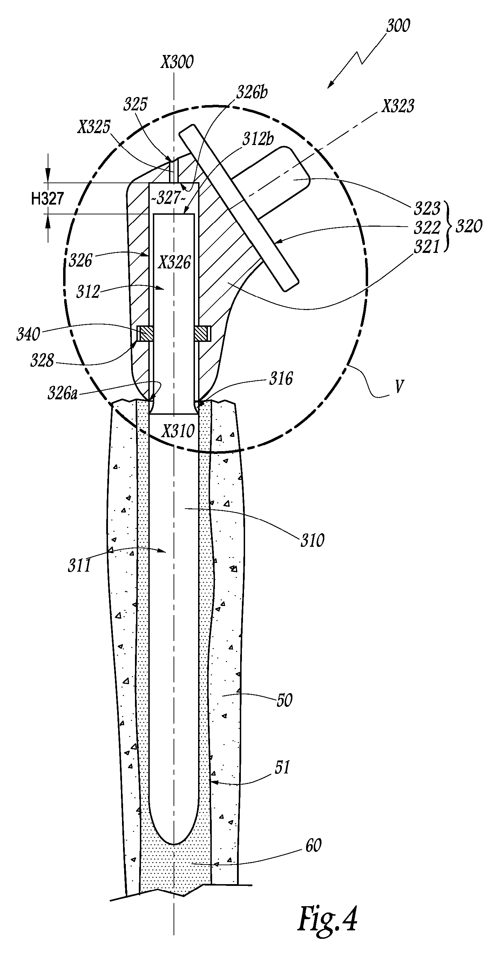

Sur les figures 4 à 6 est représenté un quatrième mode de réalisation d'une prothèse modulaire 300 conforme à l'invention. La prothèse 300 est destinée à être disposée dans le même os 50 que dans le premier mode de réalisation.

Certains éléments constitutifs de la prothèse 300 sont similaires aux éléments constitutifs de la prothèse 1 du premier mode de réalisation, décrit plus haut, et portent les mêmes références augmentées de 300. Il s'agit de la tige cylindrique 310 d'axe X310 comprenant une partie distale 311 et une partie proximale 312 avec une extrémité 312b, de l'élément métaphysaire 320, de la surface d'appui 322, du plot 323 d'axe X323, du canal d'injection 125 d'axe X125, de la cavité de couplage 326 avec un axe X326, une embouchure 326a et une surface intérieure 326b, de la chambre de réception 327, de la gorge de réception 328, ainsi que du joint annulaire 340.

Les différences se retrouvent au niveau de l'interface entre la tige 310 et l'élément métaphysaire 320, plus précisément au niveau de la partie proximale 312, la cavité 326, la chambre 327, la gorge 328 et le joint 340.

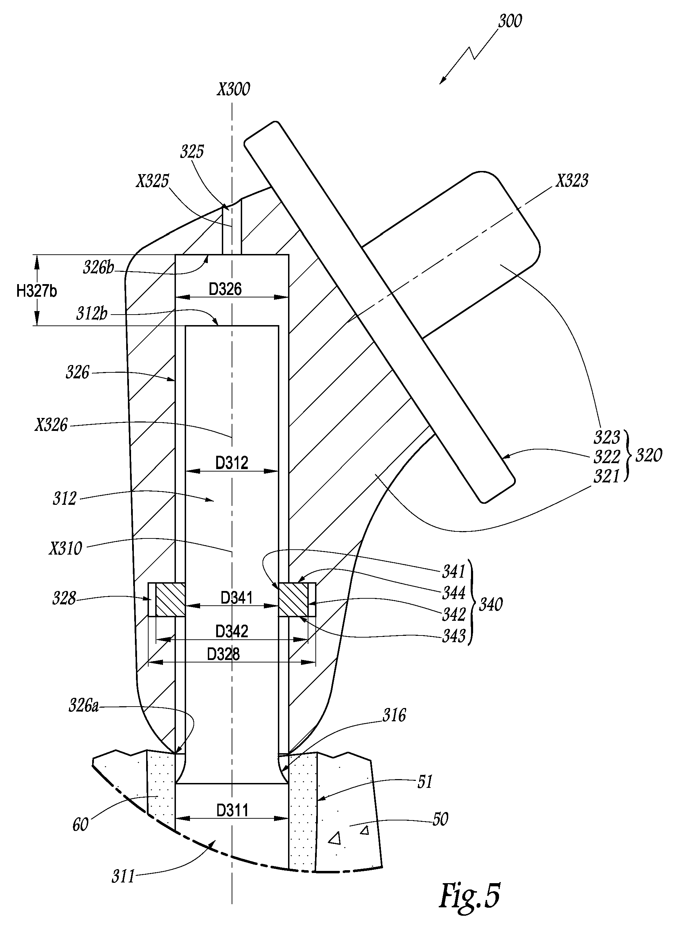

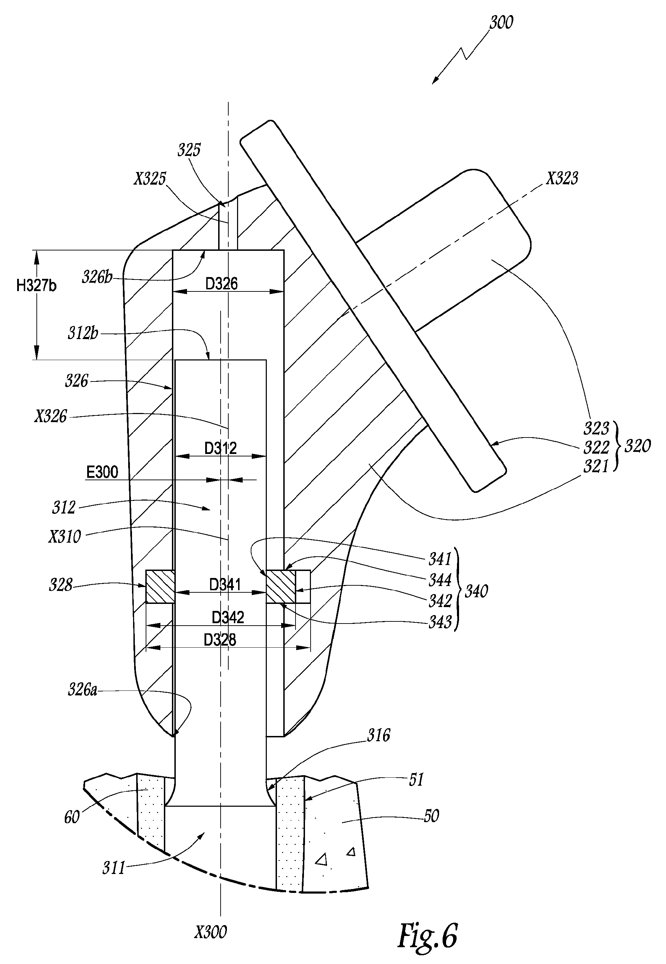

Une réduction de diamètre 316 est prévue sur la tige 310, entre la partie distale 311 qui présente un diamètre D311 et la partie proximale 312 qui présente un diamètre D312 inférieur au diamètre D311. A l'inverse du premier mode de réalisation pour lequel la partie proximale 12 de la tige 10 pénètre dans la cavité de couplage 26, avec coulissement des surfaces cylindriques complémentaires, dans ce cas un jeu transverse est ménagé entre la partie proximale 312 et la cavité 326. Ainsi, les axes X310 et X326 sont alignés sur les figures 4 et 5, et décalés transversalement sur la figure 6.

Le joint annulaire 340 comporte un alésage intérieur 341 de diamètre D341 qui est initialement inférieur au diamètre D312, une surface cylindrique extérieure 342 de diamètre D342, une surface annulaire 343 tournée vers la partie distale 311, et une surface annulaire 344 tournée vers le canal d'injection 325. Le joint 340 se déforme lorsque la partie proximale 312 est introduite en glissant dans l'alésage 341, de sorte que le diamètre D341 est sensiblement égal au diamètre D312 une fois le joint 340 positionné sur la tige 310. Les surfaces 343 et 344 sont montées glissantes perpendiculairement à l'axe X326 dans la gorge 328, qui présente un diamètre intérieur D328 supérieur au diamètre D342. Ainsi, le joint 340 réalise l'étanchéité de la chambre 327 et de la cavité 326 du côté orienté vers la diaphyse 50, tout en étant mobile, d'une part, perpendiculairement à l'axe X326 dans la gorge 328 et, d'autre part, axialement suivant l'axe X310 en restant solidaire de l'élément métaphysaire 320 et en glissant sur la partie proximale 312 de la tige 310.

En variante non représentée, la tige 310 ne présente pas de réduction de diamètre 316, tandis que la cavité 326 présente un diamètre D326 qui est supérieur au diamètre D312 de la partie proximale 312, de sorte à ménager un jeu entre elles transversalement à l'axe X300.

Selon une autre variante non représentée, la tige 310 et/ou la cavité 326 peuvent présenter respectivement d'autres configurations géométriques différentes, adaptées à la présente application.

En pratique, comme dans le précédent mode de réalisation, la tige 310 est tout d'abord insérée dans la cavité diaphysaire 51, puis bloquée dans sa configuration finale par injection de ciment ou de résine 60 à l'état liquide dans la cavité 51.

Ensuite, après séchage de la résine 60, l'élément métaphysaire 320 est positionné par le chirurgien sur la tige 310, qui fait office de support intermédiaire entre l'élément métaphysaire 320 et l'os 50. Plus précisément, la partie proximale 312 de la tige 310 pénètre dans la cavité de couplage 326 et coulisse dans l'alésage 341 du joint 340 prépositionné dans la gorge 328. Ainsi, la chambre 327, qui est délimitée par les parois de la cavité 326, la surface 344 du joint 340 et la partie proximale 312, présente un volume variable.

A ce stade, la hauteur de la prothèse 300 peut être réglée en injectant progressivement un fluide, non représenté, par le canal d'injection 325 qui débouche au niveau de la surface 326b, jusque dans la chambre de réception 327. La tige 310 est rigidement fixée dans la cavité diaphysaire 51, tandis que l'élément métaphysaire 320 peut se déplacer relativement à la partie proximale 312. Sous l'effet de la pression interne à la chambre 327, exercée par le fluide entre la cavité 326 et la partie proximale 312, plus précisément entre les surfaces 312b et 326b, le volume de la chambre 327 augmente.

En particulier, la chambre de réception 327 présente une dimension H327b variable suivant la direction longitudinale de la prothèse 300, définie par son axe X300, lequel est confondu avec l'axe X310 de la tige 310. La dimension H327b peut être mesurée suivant l'axe X300 entre les surfaces 312b et 326b. Autrement dit, la dimension H327b de la chambre de réception 327 de fluide est variable, en fonction de la quantité de fluide admise dans la chambre 327, suivant l'axe X300. De ce fait, lorsque le fluide est injecté, la hauteur de la prothèse 300 augmente progressivement jusqu'à atteindre la position désirée.

Par ailleurs, comme visible à la figure 6, l'élément métaphysaire 320 peut être décalé latéralement par rapport à la tige 310 afin de positionner au mieux la prothèse 300. Dans ce cas, un écart transverse E300 apparaît entre les axes X31 0 et X326, c'est-à-dire que cet écart E300 est mesuré dans un plan perpendiculaire aux axes X310 et X326. Le joint 340 est alors déplacé dans la gorge 328, de manière que l'alésage 341 maintienne l'étanchéité sur la partie proximale 312, tandis que les surfaces 343 et 344 maintiennent sur un rayon donné de son diamètre D342, au fond de la gorge 328.

En variante non représentée, la gorge 328 présente un diamètre D328 plus important. Dans ce cas, la surface 342 ne vient pas en butée au fond de la gorge 328, et la partie proximale 312 vient en contact avec la paroi cylindrique de la cavité 326.

En d'autres termes, la chambre 327 présente un volume variable en fonction de la longueur de tige 310 insérée dans la cavité 326 et de la quantité de fluide introduite dans la chambre 327. Egalement, pour un même volume interne, la chambre 327 présente une configuration variable en fonction de l'écart E300 entre les axes X312 et X326.

Selon une variante préférée, le fluide peut être un fluide agglutinant. Ainsi, le positionnement relatif entre les éléments 310 et 320 est variable et peut être réglé avec précision, avant, pendant ou après l'injection du fluide. Ce positionnement est variable axialement suivant l'axe longitudinal X300 de la prothèse et/ou transversalement à cet axe longitudinal X300 et/ou angulairement autour de cet axe longitudinal X300.

Selon une variante particulière non représentée, la prothèse peut comprendre plusieurs chambres de réception de fluide agglutinant. De préférence, les chambres délimitées à l'intérieur de la prothèse sont isolées les unes des autres, de sorte que chaque chambre peut être remplie indépendamment des autres chambres. Par exemple, les chambres peuvent être délimitées par des parois de séparation qui sont radiales ou concentriques par rapport à l'axe longitudinal de la prothèse. Les différentes chambres peuvent présenter différentes configurations adaptées à la présente application. A titre d'exemple pratique non limitatif, une première chambre peut être remplie de fluide lors de la mise en place initiale de la prothèse, puis une deuxième chambre peut être remplie de fluide lors d'une reprise de la prothèse afin de modifier le positionnement relatif des éléments métaphysaires et diaphysaires.

En pratique, la prothèse modulaire selon l'invention permet de réparer une fracture, sans nécessiter d'instrumentation encombrante et difficile à manipuler pour ajuster la prothèse, en particulier pour ajuster le positionnement relatif entre l'élément métaphysaire et l'élément diaphysaire. De ce fait, la prothèse permet de s'affranchir d'un ancillaire et d'implants d'essais. La prothèse peut être facilement ajustée en hauteur et/ou latéralement et/ou angulairement, puis être rendue monobloc et verrouillée en place à l'aide du fluide agglutinant lorsque le réglage est satisfaisant, sans qu'il soit nécessaire d'utiliser des données issues d'essais préliminaires multiples effectuées sur la prothèse.

Egalement, la prothèse selon l'invention peut être mise en oeuvre dans le cadre particulier d'une reprise chirurgicale et/ou dans le cadre de toute application post-opératoire. Dans ce cas, le chirurgien désolidarise l'élément diaphysaire et l'élément métaphysaire en introduisant du fluide sous pression dans la chambre de réception. Par exemple, la prothèse peut être configurée pour recevoir une seringue d'injection de fluide. Ce fluide peut être un fluide agglutinant, ou bien un fluide « désagglutinant », ce qui permet de repositionner les éléments de prothèse avant de procéder à une nouvelle injection de fluide agglutinant.

En outre, les caractéristiques techniques des différents modes de réalisation peuvent être, en totalité ou pour certaines d'entre elles, combinées entre elles. Ainsi, la prothèse peut être adaptée aux besoins particuliers du chirurgien. Avantageusement, la prothèse peut être mise en oeuvre avec tous types d'éléments métaphysaires, anatomiques ou inversés.

En pratique, le chirurgien peut disposer d'un kit chirurgical comprenant différents éléments de prothèse modulaire. De préférence, un tel kit prothétique modulaire inclut différents éléments métaphysaires correspondant à différentes hauteurs de tête et/ou différentes configurations de cavité(s). Par ailleurs, le kit peut inclure différentes tiges diaphysaires, correspondant à différentes hauteurs et/ou différents diamètres de tige proximale. Enfin, le kit peut inclure un récipient comprenant une quantité donnée de fluide agglutinant, suffisante pour l'implantation d'au moins une prothèse. Le kit est à disposition du chirurgien sous forme d'une boite, coffret ou emballage, connu en soi, dont l'agencement peut être prévu pour faciliter le repérage des éléments modulaires et faire gagner du temps au chirurgien. Différents kits sont envisageables, proposant un nombre variable d'éléments modulaires, soit réduit et donc moins coûteux, soit important et donc adapté à un grand nombre de cas.

Ainsi, grâce à un tel kit prothétique modulaire, le chirurgien peut faire face rapidement à toutes les situations qui peuvent se présenter lors de l'opération chirurgicale.

## Revendications

1. Prothèse modulaire (1 ; 100 ; 200 ; 300), comprenant au moins un premier élément (10 ; 110 ; 210 ; 310) et un deuxième élément (20 ; 120 ; 320), **caractérisée en ce qu'**au moins une chambre de réception (27 ; 127 ; 327) d'un fluide est délimitée à l'interface entre le premier élément (10 ; 110 ; 210 ; 310) et le deuxième élément (20 ; 120 ; 320).

2. Prothèse modulaire selon la revendication 1, **caractérisée en ce que** chaque chambre de réception (27 ; 127 ; 327) présente un volume variable, notamment en fonction de la quantité de fluide admise dans la chambre (27 ; 127 ; 327).

3. Prothèse modulaire selon l'une des revendications précédentes, **caractérisée en ce que** plusieurs chambres de réception (27 ; 127 ; 327) isolées sont délimitées à l'intérieur de la prothèse (1 ; 100 ; 200 ; 300), ces chambres de réception étant aptes à être remplies indépendamment les unes des autres.

4. Prothèse modulaire selon l'une des revendications précédentes, **caractérisée en ce que** le positionnement relatif entre le premier élément (10 ; 110 ; 210 ; 310) et le deuxième élément (20 ; 120 ; 320) est variable axialement suivant un axe longitudinal (X1 ; X100 ; X21 0 ; X300) de la prothèse (1 ; 100 ; 200 ; 300) et/ou transversalement à cet axe longitudinal (X300) et/ou angulairement autour de cet axe longitudinal (X1 ; X100 ; X300).

5. Prothèse modulaire selon l'une des revendications précédentes, **caractérisée en ce que** l'un des éléments (10 ; 20 ; 110 ; 120 ; 210 ; 310 ; 320) parmi le premier élément et le deuxième élément inclut au moins un canal d'injection (25 ; 125 ; 325) pour l'injection du fluide dans la ou les chambres de réception (27 ; 127 ; 327).

6. Prothèse modulaire selon l'une des revendications précédentes, **caractérisée en ce que** le deuxième élément (20 ; 120 ; 320) inclut au moins une cavité de couplage (26 ; 126 ; 326) apte à recevoir au moins partiellement le premier élément (10 ; 110 ; 210 ; 310), et **en ce que** la ou les chambres de réception (27 ; 127 ; 327) sont délimitées dans la cavité de couplage (26 ; 126 ; 326).

7. Prothèse modulaire (200) selon la revendication 6, **caractérisée en ce qu'**une partie proximale (212) du premier élément (210) et la cavité de couplage (26 ; 126 ; 326) du deuxième élément (20 ; 120 ; 320) comprennent des moyens (213) de blocage complémentaires, qui sont aptes à coopérer et bloquer le déplacement relatif en rotation entre le premier élément (210) et le deuxième élément (20 ; 120 ; 320), notamment des rainures, cannelures, dentures et/ou aspérités de surface.

8. Prothèse modulaire (1 ; 100 ; 200) selon l'une des revendications 6 ou 7, **caractérisée en ce que** l'un des éléments (10 ; 20 ; 110 ; 120 ; 210 ; 310 ; 320) parmi le premier élément et le deuxième élément inclut au moins un canal d'injection (25 ; 125 ; 325) pour l'injection du fluide dans la ou les chambres de réception (27 ; 127 ; 327), et **en ce que** le canal d'injection (25 ; 125), la cavité de couplage (26 ; 126) et le premier élément (10 ; 110 ; 210) sont coaxiaux.

9. Prothèse modulaire (200) selon l'une des revendications précédentes, **caractérisée en ce qu'**un épaulement (214) est agencé entre une partie distale (211) et une partie proximale (212) du premier élément (210).

10. Prothèse modulaire selon l'une des revendications précédentes, **caractérisée en ce que** l'un des éléments (10 ; 20 ; 110 ; 120 ; 210 ; 310 ; 320) parmi le premier élément et le deuxième élément inclut une gorge de réception (28 ; 128 ; 328) annulaire, du côté de la ou les chambres de réception (27 ; 127 ; 327) qui est orienté vers l'autre élément (10 ; 110 ; 310), et **en ce qu'**un joint d'étanchéité (40 ; 140 ; 340) annulaire est disposé dans la gorge de réception (28 ; 128 ; 328) de manière à réaliser l'étanchéité de la ou des chambres de réception (27 ; 127 ; 327) de fluide du côté tourné vers l'autre élément.

11. Prothèse modulaire selon l'une des revendications précédentes, **caractérisée en ce que** la ou au moins l'une des chambres de réception (27 ; 127 ; 327) est configurée pour recevoir un fluide agglutinant à l'interface entre le premier élément (10 ; 110 ; 210 ; 310) et le deuxième élément (20 ; 120 ; 320), notamment au contact direct du premier élément et du deuxième élément.

12. Prothèse modulaire selon l'une des revendications précédentes, **caractérisée en ce que** la prothèse (1 ; 100 ; 200 ; 300) comprend un fluide agglutinant remplissant la ou l'une des chambres de réception (27 ; 127 ; 327).

13. Prothèse modulaire selon l'une des revendications 1 à 12, **caractérisée en ce que** le fluide est un fluide agglutinant choisi parmi les matériaux suivants : polyméthacrylate de méthyle, résine bio-compatible, matériau adhésif, matériau polymère, ciment chirurgical, substitut osseux injectable, et de préférence le temps de séchage du fluide agglutinant est compris entre 5 et 30 minutes.

14. Prothèse modulaire selon l'une des revendications 1 à 12, **caractérisée en ce que** le fluide est non-agglutinant et choisi parmi de l'air, de l'eau, une solution liquide ou un gel aqueux.

15. Kit chirurgical, **caractérisé en ce qu'**il comprend au moins une prothèse modulaire (1 ; 100 ; 200 ; 300) selon l'une des revendications précédentes, avec :
- au moins un premier élément (10 ; 110 ; 210 ; 310) et
- au moins deux deuxièmes éléments (20 ; 120 ; 320), chaque deuxième élément présentant des dimensions différentes du ou des autres deuxièmes éléments et étant configuré pour former au moins une chambre (27 ; 127 ; 327) de dimensions différentes à l'interface avec le ou l'un des premiers éléments.
